# EUROPÄISCHE PATENTSCHRIFT

(11) **EP 1 588 689 B1**
(45) Veröffentlichungstag und Bekanntmachung des Hinweises auf die Patenterteilung: **20.09.2006**
(21) Anmeldenummer: 05003963.5
(22) Anmeldetag: 24.02.2005
(51) Int. Cl.: A61Q 5/06, A61Q 5/10, A61K 8/81

(54) **Itaconsäuremonoester/Acrylat Copolymer und Polystyrolsulfonat enthaltende Haarbehandlungsmittel**
Hair treatments comprising an itaconic acid monoester /acrylate copolymer and polystyrenesulfonate.
Agents de traitement capillaire comprenant un copolymère acrylique-itaconique estérifié.

(30) Priorität: 17.04.2004 DE 102004018723
(43) Veröffentlichungstag der Anmeldung: 26.10.2005
(73) Patentinhaber: Wella Aktiengesellschaft, 64274 Darmstadt (DE)
(72) Erfinder: Walter, Andrea, 64572 Büttelborn (DE); Birkel, Susanne, Dr., 64285 Darmstadt (DE); Hannich, Manuela, 63329 Egelsbach (DE); Kalbfleisch, Axel, 64295 Darmstadt (DE)

(56) Entgegenhaltungen:
- EP-A- 1 287 810
- WO-A-02/098386
- DE-A1- 10 032 118
- DE-A1- 19 921 962
- PATENT ABSTRACTS OF JAPAN Bd. 2002, Nr. 11, 6. November 2002 (2002-11-06) & JP 2002 193772 A (HOYU CO LTD), 10. Juli 2002 (2002-07-10)
- ANONYMOUS: "STRUCTURE 2001 / STRUCTURE 3001 [Technical bulletin]"[Online] XP002209084 Gefunden im Internet: URL:HTTP://WWW.PERSONALCAREPOLYMERS.COM/SI TE/LIST.ASP?ID=ENHANCE.7> DWWW- 2002-08-08> [gefunden am 2002-08-08]

## Beschreibung

Gegenstand der Erfindung ist ein Haarbehandlungsmittel mit einem Gehalt an mindestens einem Itaconsäuremonoester/Acrylat Copolymer und mindestens einem Polystyrolsulfonat eines ausgewählten Molekulargewichts.

Haargele werden eingesetzt, um dem menschlichen Haar Festigung und Halt zu geben. Übliche Haargele enthalten in der Regel eine Kombination aus Gelbildnern und haarfestigenden Polymeren. Die für diese Zwecke üblicherweise verwendeten kosmetischen, haarfestigenden Polymere zeigen in wässrigen, alkoholischen oder wässrig-alkoholischen Medien gute Festigungseigenschaften, die nach der Anwendung die Frisur in Form halten und festigen. In der EP 1 287 810 werden Haarbehandlungsmittel in Gelform beschrieben mit einem Gehalt an einer Kombination aus mindestens einem Itaconsäuremonoester/Acrylat Copolymer und mindestens einem haarfestigenden und filmbildenden nichtionischen, anionischen, amphoteren oder zwitterionischen Polymer. Die bekannten Gele sind noch nicht in jeder Hinsicht völlig zufriedenstellend, insbesondere hinsichtlich der Klebephase während der Einarbeitung in das Haar, den Anwendungseigenschaften auf feinem Haar hinsichtlich Belastung und Volumenwirkung sowie Klebegefühl und Verteilbarkeit in den Händen. Eine besondere Rolle spielt dabei die sogenannte Klebephase. Während und nach der Einarbeitungszeit in das Haar verdunstet das Lösungsmittel, wobei die Klebrigkeit zunächst stark zunimmt und dann in eine starke Festigung übergeht. Ein ideales Gel sollte eine angemessene, optimierte Einarbeitungszeit ermöglichen, wobei die Frisur lange genug flexibel und modellierbar bleibt bevor das Gel getrocknet ist und in eine starke Festigung übergeht. Ein ideales Gel sollte außerdem möglichst wenig an den Fingern und Händen kleben aber trotzdem gleichzeitig eine gute Haftung am Haar aufweisen. Wünschenswert sind daher Haarstylinggele, welche einen oder mehrere weitere Vorteile aufweisen ohne den guten Halt zu beeinträchtigen, z.B. feines Haar weniger belasten, eine gute Volumenbildung in feinem und kräftig-schwerem Haar bewirken, eine optimierte Klebephase und eine angemessene Einarbeitungszeit aufweisen oder bei guter Haftung am Haar ein möglichst geringes Klebegefühl an Fingern und Händen hinterlassen.

Es wurde nun gefunden, dass verbesserte Haarstylinggele zur Verfügung gestellt werden können durch ein Haarbehandlungsmittel mit einem Gehalt an einer Kombination von
(A) mindestens einem Copolymeren aus einer ersten Monomerart, ausgewählt aus Itaconsäuremonoestern der allgemeinen Formel CH₂=C(COOR¹)CH₂COOR² wobei einer der Substituenten R¹ und R² für Wasserstoff und der andere für die Gruppe -(CH₂CH₂O)ₓ-R³ steht, x eine Zahl zwischen 1 und 100 bedeutet und R³ eine Alkylgruppe mit 8 bis 30 C-Atomen bedeutet und einer zweiten Monomerart, ausgewählt aus Acrylat-Monomeren und
(B) mindestens einem Polystyrolsulfonat mit einem Molekulargewicht im Bereich von 50.000 bis 200.000.

Das Haarbehandlungsmittel liegt vorzugsweise in Gelform auf wässriger Basis vor und weist vorzugsweise eine Viskosität von mindestens 1000 mPa s bei 25°C auf.

Das Copolymer (A) wird vorzugsweise in einer Menge von 0,1 bis 30, besonders bevorzugt von 0,5 bis 15 oder von 1 bis 10 Gew.% eingesetzt. Das Polystyrolsulfonat wird vorzugsweise in einer Menge von 0,1 bis 20, besonders bevorzugt von 0,5 bis 10 oder von 1 bis 7 Gew.% eingesetzt. Das erfindungsgemäße Haarbehandlungsmittel ist vorzugsweise im wesentlichen frei von kationischen Polymeren, d.h. es enthält entweder keine oder weniger als 0,5, vorzugsweise weniger als 0,1 Gew.% an kationischen Polymeren.

Das erfindungsgemäße Haargel bewirkt insbesondere eine gute Definition und eine langanhaltende Texture von kurzem Haar und gibt einen sehr schönen Glanz. Das Gel ist gut in der Hand verteilbar und hat eine gute Klebephase, die lange genug anhält, um die gewünschte Frisur modellieren zu können, bevor das Gel fest wird.

### Copolymer (A)

Das Copolymer (A) ist aufgebaut aus Itaconsäuremonoestern der allgemeinen Formel CH₂=C(COOR¹)CH₂COOR² wobei einer der Substituenten R¹ und R² für Wasserstoff und der andere für die Gruppe -(CH₂CH₂O)ₓ-R³ steht. X ist eine Zahl zwischen 1 und 100 vorzugsweise zwischen 10 und 40, besonders bevorzugt 20. R³ ist eine Alkylgruppe mit 8 bis 30, vorzugsweise 12 bis 20 C-Atomen, z.B. Cetyl oder Stearyl, wobei Cetyl besonders bevorzugt ist.

Die Acrylat-Monomeren des Copolymers (A) sind vorzugsweise ausgewählt aus Acrylsäure, Methacrylsäure und deren einfachen Estern, insbesondere den Acrylsäurealkylestern und Methacrylsäurealkylestern mit 1 bis 10, vorzugsweise 1 bis 4 C-Atomen in der Alkylgruppe. Geeignete Copolymere sind beispielsweise Acryl- oder Methacrylsäure/Itaconsäurepolyethoxyalkylester Copolymere (INCI-Bezeichnungen: Acrylates/Steareth-20 Itaconate Copolymer und Acrylates/Ceteth-20 Itaconate Copolymer), wie sie von der Firma National Starch/USA unter den Bezeichnungen Structure® 2001 und Structure® 3001 vertrieben werden. Die Säuregruppen sind in den eingesetzten Polymeren vorzugsweise durch organische oder anorganische Basen zu 50 bis 100% neutralisiert. Geeignete Neutralisationsmittel sind primäre oder sekundäre Amine, insbesondere Aminoalkanole mit vorzugsweise 1 bis 10 C-Atomen und 1 bis 3 Hydroxygruppen wie z.B. Aminomethylpropanol (AMP), Triethanolamin, Tetrahydroxypropylethylendiamin oder Monoethanolamin, aber auch Ammoniak, NaOH, KOH u.a..

In einer besonderen Ausführungsform sind in dem erfindungsgemäßen Mittel mindestens ein erstes und mindestens ein zweites Copolymer aus jeweils einem Itaconsäuremonoester der Formel CH₂=C(COOH)CH₂COO-(CH₂CH₂O)ₓ-R³ und Acrylatmonomeren enthalten, wobei x eine Zahl zwischen 10 und 40 und R³ des ersten Copolymers eine Cetylgruppe und R³ des zweiten Copolymers eine Stearylgruppe bedeuten. Das Gewichtsverhältnis von erstem Copolymer (Cetylderivat) zu zweitem Copolymer (Stearylderivat) ist vorzugwseise größer oder gleich 1:1, insbesondere von 2:1 bis 10:1, besonders bevorzugt von 3:1 bis 5:1. Zusammensetzungen mit einer Kombination von zwei Itaconatcopolymeren zeichnen sich durch eine besondere Langzeitstabilität der besonders vorteilhaften Konsistenz und rheologischen Eigenschaften aus.

### Polystyrolsulfonat (B)

Das Polystyrolsulfonat ist herstellbar aus Polymerisierung von Styrol, Sulfonierung des erhaltenen Polystyrols und Salzbildung aus der erhaltenen Poly(styrolsulfonsäure) mit einer geeigneten Base, z.B. Natriumhydroxid. Eine typische allgemeine Formel ist -[CH(C6H4-SO₃⁻ A⁺)-CH₂]ₙ-
wobei n eine natürliche Zahl größer Null ist, welche den Polymerisationsgrad angibt und so gewählt ist, dass das Molekulargewicht des Polymers im Bereich von 50.000 bis 200.000, vorzugsweise von 80.000 bis 180.000 liegt. A⁺ ist ein geeignetes Kation, insbesondere ein Metallkation, vorzugsweise ein Alkali oder Erdalkalikation, z.B. Natrium. Ein geeignetes Handelsprodukt ist z.B. Flexan® 130 oder Flexan® II mit einem Molekulargewicht von etwa 130.000, einer Glasübergangstemperatur von etwa 112°C und einer Viskosität im Bereich von 50 bis 250 cps (30%ige wässrige Lösung, RTV Brookfield Viskosimeter, 20 U/min, Spindel Nr. 2, 25°C).

In einer besonderen Ausführungsform enthält das erfindungsgemäße Mittel zusätzlich mindestens ein weiteres, von den Polymeren (A) und (B) verschiedenes, haarfestigendes Polymer. Das zusätzliche haarfestigende Polymer kann nichtionisch, anionisch, zwitterionisch oder amphoter sein ist aber vorzugsweise nichtionisch oder anionisch. Es kann ein synthetisches oder ein natürliches Polymer sein. Unter natürlichen Polymeren werden auch chemisch modifizierte Polymere natürlichen Ursprungs verstanden. Bevorzugt sind insbesondere solche Polymere, die eine ausreichende Löslichkeit in Wasser, Alkohol oder Wasser/Alkohol-Gemischen besitzen, um in dem erfindungsgemäßen Mittel in vollständig gelöster Form vorzuliegen. Unter haarfestigenden Polymeren werden erfindungsgemäß solche Polymere verstanden, die bei Anwendung in 0,01 bis 5%iger wässriger, alkoholischer oder wässrig-alkoholischer Lösung oder Dispersion in der Lage sind, auf dem Haar eine haarfestigende Wirkung zu erzielen. Haarfestigende Polymere sind insbesondere solche Polymere, für die im International Cosmetic Ingredient Dictionary and Handbook als Funktion "Hair Fixatives" angegeben ist.

Geeignete synthetische, nichtionische haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Dialkylaminoalkylmethacrylamid, Dialkylaminoalkylacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids. Weitere geeignete haarfestigende Polymere sind z.B. Copolymerisate aus Vinylpyrrolidon und Vinylacetat, Terpolymere aus Vinylpyrrolidon, Vinylacetat und Vinylpropionat, Terpolymere aus Vinylpyrrolidon, Vinylcaprolactam und Dialkylaminoalkyl(meth)acrylat, Terpolymere aus Vinylpyrrolidon, Vinylcaprolactam und Dialkylaminoalkyl(meth)-acrylamid, Polyacrylamide, Polyvinylalkohole, sowie haarfestigende Polyethylenglykol/Polypropylenglykol Copolymere.

Besonders bevorzugte nichtionische Polymere sind Polyvinylpyrrolidon und Polyvinylpyrrolidon/Vinylacetat Copolymere.

Geeignete anionische haarfestigende Polymere können natürliche oder synthetische Homo- oder Copolymere mit Säuregruppen enthaltenden Monomereinheiten sein, welche gegebenenfalls mit Comonomeren, die keine Säuregruppen enthalten, copolymerisiert sind. Die Säuregruppen sind vorzugsweise ausgewählt aus -COOH, -SO₃H, -OSO₃H, -OPO₂H und -OPO₃H₂, von denen die Carbonsäuregruppen bevorzugt sind. Die Säuregruppen können unneutralisiert, teilweise oder vollständig neutralisiert vorliegen. Sie liegen vorzugsweise zu 50 bis 100% in anionischer bzw. neutralisierter Form vor. Als Neutralisationsmittel können die oben genannten Neutralisationsmittel verwendet werden. Geeignete Monomere sind ungesättigte, radikalisch polymerisierbare Verbindungen, welche mindestens eine Säuregruppe tragen, insbesondere Carboxyvinylmonomere. Geeignete Säuregruppen enthaltende Monomere sind beispielsweise Acrylsäure, Methacrylsäure, Crotonsäure, Maleinsäure bzw. Maleinsäureanhydrid oder deren Monoester, Aldehydocarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie z.B. Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete anionische Polymere sind insbesondere Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte anionische Polymere sind vernetzte oder unvernetzte Vinylacetat/Crotonsäure Copolymere. Ebenso bevorzugt sind partialveresterte Copolymere zwischen Vinylmethylether und Maleinsäureanhydrid. Weitere geeignete anionische Polymere sind z.B. Terpolymere aus Acrylsäure, Alkylacrylat und N-Alkylacrylamid, insbesondere Acrylsäure/Ethylacrylat/N-t-Butylacrylamid Terpolymere oder Terpolymere aus Vinylacetat, Crotonat und Vinylalkanoat, insbesondere Vinylacetat/Crotonat/Vinylneodecanoat Copolymere.

Geeignete filmbildende amphotere Polymere, sind Polymere, welche neben sauren oder anionischen Gruppen als weitere funktionelle Gruppen basische oder kationische Gruppen, insbesondere primäre, sekundäre, tertiäre oder quaternäre Amingruppen enthalten. Beispiele hierfür sind Copolymere gebildet aus Alkylacrylamid (insbesondere Octylacrylamid), Alkylaminoalkylmethacrylat (insbesondere t-Butylaminoethylmethacrylat) und zwei oder mehr Monomeren ausgewählt aus Acrylsäure, Methacrylsäure oder deren Ester, wobei die Alkylgruppen 1 bis 4 C-Atome enthalten, mindestens eines der Monomere eine Säuregruppe aufweist und wie sie z.B. unter dem Handelsnamen Amphomer® oder Amphomer® LV-71 der Firma NATIONAL STARCH, USA erhältlich sind.

Weitere Beispiele für geeignete haarfestigende Polymere sind Copolymere von Acrylsäure, Methylacrylat und Methacrylamidopropyltrimethylammoniumchlorid (INCI-Bezeichnung: Polyquaternium-47), Copolymere aus Acrylamidopropyltrimethylammoniumchlorid und Acrylaten, oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43). Geeignet sind auch Polymere mit Betaingruppen tragenden Monomeren wie z.B. Copolymere aus Methacryloylethylbetain und zwei oder mehr Monomeren von Acrylsäure oder deren einfachen Estern, bekannt unter der INCI-Bezeichnung Methacryloyl Ethyl Betaine/Acrylates Copolymer.

Das erfindungsgemäße Mittel wird bevorzugt in einem wässrigen, einem alkoholischen oder in einem wässrig-alkoholischen Medium mit vorzugsweise mindestens 10 Gew.% Wasser und vorzugsweise maximal 40 Gew.% Alkohol konfektioniert. Als Alkohole können insbesondere die für kosmetische Zwecke üblicherweise verwendeten niederen Monoalkohole mit 1 bis 4 Kohlenstoffatomen wie z.B. Ethanol und Isopropanol enthalten sein. Das erfindungsgemäße Mittel weist einen pH-Wert von vorzugsweise 6 bis 9 auf. Besonders bevorzugt ist ein pH-Wert größer 7, insbesondere größer 7,3.

In einer bevorzugten Ausführungsform enthält das Gel zur weiteren Glanzverbesserung mehrwertige Alkohole, vorzugsweise solche mit 2 bis 5 C-Atomen und mit 2 bis 5 Hydroxygruppen in einer Menge von 0,1 bis 15, bevorzugt von 1 bis 10 Gew.%. Besonders bevorzugt sind Glycerin, Ethylenglykol, Propylenglykol und Pentandiol, insbesondere 1,2-Propylenglykol und 1,2-Pentandiol.

Vorzugsweise liegt das erfindungsgemäße Haarbehandlungsmittel in Form eines Gels vor. Die Viskosität des Gels beträgt vorzugsweise von 1000 bis 100.000, besonders bevorzugt von 2000 bis 50.000 mPa s, ganz besonders bevorzugt von 2.500 bis 15.000 mPa s, gemessen als dynamische Viskositätsmessung mit einem HAAKE VT-550 Rheometer, Messkörper SV-DIN bei einer Temperatur von 25°C und einer Schergeschwindigkeit von 50 s⁻¹.

Das erfindungsgemäße Mittel kann darüber hinaus die für Haarbehandlungsmittel üblichen Zusatzbestandteile enthalten, z.B. Netzmittel oder Emulgatoren aus den Klassen der nichtionischen, anionischen, kationischen oder amphoteren oberflächenaktiven Tenside, wie Fettalkoholsulfate, Alkylbenzolsulfonate, Alkyltrimethylammoniumsalze, Alkylbetaine, ethoxylierten Fettalkoholen, ethoxylierten Fettsäureglyceriden, in einer Menge von 0,1 bis 15 Gew.%; Feuchthaltemittel; Parfümöle in einer Menge von 0,1 bis 1 Gew.%; Trübungsmittel, wie z.B. Ethylenglykoldistearat, in einer Menge von etwa 0,2 bis 5,0 Gew.%; Perlglanzmittel, wie z.B. ein Gemisch aus Fettsäuremonoalkylolamid und Ethylenglykoldistearat, in einer Menge von etwa 1,0 bis 10 Gew.%; bakterizide und fungizide Wirkstoffe wie z.B. 2,4,4-Trichlor-2-hydroxydiphenylether oder Methylchlorisothiazolinon, in einer Menge von 0,01 bis 1,0 Gew.%; Verdickungsmittel, wie z.B. Kokosfettsäurediethanolamid, in einer Menge von etwa 0,2 bis 3,0 Gew.%, Puffersubstanzen, wie z.B. Natriumcitrat oder Natriumphosphat, in einer Menge von 0,1 bis 1,0 Gew.%; Anfärbestoffe, wie z.B. Fluorescein Natriumsalz, in einer Menge von etwa 0,1 bis 1,0 Gew.%; Pflegestoffe, wie z.B. Pflanzen- und Kräuterextrakte, Protein- und Seidenhydrolysate, Lanolinderivate, in einer Menge von 0,1 bis 5 Gew.%; physiologisch verträgliche Silikonderivate, wie z.B. flüchtige oder nicht-flüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe, in einer Menge von etwa 0,01 bis 4 Gew.%; Fettalkohole, Glanzgeber, Vitamine, Weichmacher, Kämmbarkeitsverbesserer, rückfettende Agenzien und Entschäumer.

Das erfindungsgemäße Mittel wird vorzugsweise in Form eines klaren, transparenten oder zumindest durchscheinenden Gels eingesetzt, wobei das Gel farblos oder eingefärbt sein kann.

Das erfindungsgemäße Mittel zeichnet sich durch seine besonderen rheologischen Eigenschaften aus, die sich insbesondere auch in einer attraktiven, besonders ansprechenden Haptik manifestieren. Das Mittel ist gut auf Haaren verteilbar. Es wirkt nicht belastend auf das Haar und ist daher insbesondere auch für feines Haar geeignet. Es bewirkt eine gute Haltbarkeit der Frisur, ohne dass das Haar verklebt oder belastet wird. Feines Haar bekommt Fülle und Volumen. Als haarfestigendes Gel zeigt es gute festigende Eigenschaften und insbesondere einen starken Glanz.

In einer besonderen Ausführungsform ist das erfindungsgemäße Mittel zur gleichzeitigen Festigung und temporären Haarfärbung geeignet und enthält zusätzlich mindestens ein temporär haarfärbendes Pigment. Unter temporärer Haarfärbung wird eine Farbänderung von menschlichen Haaren verstanden, die bis zur nächsten Haarwäsche hält und durch Waschen der Haare mit üblichen Shampoos wieder entfernt werden kann. Die Pigmente sind vorzugsweise in einer Menge von 0,01 bis 25 Gew.%, besonders bevorzugt in einer Menge von 5 bis 15 Gew.% enthalten. Bei den Pigmenten handelt es sich vorzugsweise nicht um Nano- sonder um Mikropigmente. Die bevorzugte Teilchengröße beträgt 1 bis 200 µm, insbesondere 3 bis 150 µm, besonders bevorzugt 10 bis 100 µm.

Die Pigmente sind im Anwendungsmedium praktisch unlösliche Farbmittel und können anorganisch oder organisch sein. Auch anorganisch-organische Mischpigmente sind möglich. Bevorzugt sind anorganische Pigmente. Der Vorteil der anorganischen Pigmente ist deren ausgezeichnete Licht-, Wetter- und Temperaturbeständigkeit. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Buntpigmente wie z.B. Ultramarin oder Eisenoxidrot, um Glanzpigmente, Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist. Geeignet sind Metalloxide, -hydroxide und -oxidhydrate, Mischphasenpigmente, schwefelhaltige Silicate, Metallsulfide, komplexe Metallcyanide, Metallsulfate, -chromate und -molybdate sowie die Metalle selbst (Bronzepigmente). Geeignet sind insbesondere Titandioxid (CI 77891), schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510), Carmine (Cochineal).

Besonders bevorzugt sind Pigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt sein kann. Derartige Pigmente werden beispielsweise unter den Handelsbezeichnung Rona® , Colorona®, Dichrona® und Timiron® von der Firma Merck, Deutschland vertrieben.

Organische Pigmente sind beispielsweise die natürlichen Pigmente Sepia, Gummigutt, Knochenkohle, Kasseler Braun, Indigo, Chlorophyll und andere Pflanzenpigmente. Synthetische organische Pigmente sind beispielsweise Azo-Pigmente, Anthrachinoide, Indigoide, Dioxazin-, Chinacridon-, Phtalocyanin-, Isoindolinon-, Perylen- und Perinon-, Metallkomplex-, Alkaliblau- und Diketopyrrolopyrrol-Pigmente.

Eine bevorzugte Ausführungsform betrifft ein Haarbehandlungsmittel mit einem Gehalt an einer Kombination von
(A) 0,5 bis 15 Gew.% eines Copolymeren aus einer ersten Monomerart, ausgewählt aus ethoxylierten Itaconsäuremonocetylestern mit einem Ethoxylierungsgrad von 10 bis 40 und einer zweiten Monomerart, ausgewählt aus Acrylat-Monomeren und
(B) 0,5 bis 7 Gew.% eines Polystyrolsulfonats mit einem Molekulargewicht von 80.000 bis 180.000,
wobei das Mittel in Form eines wässrigen Gels vorliegt und einen pH-Wert von 6 bis 9 aufweist.

Die nachfolgenden Beispiele sollen den Gegenstand der Erfindung näher erläutern. Die in den Beispielen angegebenen Polymergehalte beziehen sich jeweils auf den Feststoffgehalt, sofern nichts anderes angegeben ist.

### Beispiele

### Beispiel 1: Haargel

| | |
|---|---|
| 10 g | Structure® 3001 ¹⁾ |
| 5 g | Flexan® II ²⁾ |
| 5 g | 1,2-Propylenglykol |
| 0,2 g | PEG-40 hydriertes Rizinusöl |
| 0,2 g | Parfüm |
| Ad 100 | Wasser |

| | |
|---|---|
| ¹⁾Acrylates/Ceteth-20 Itaconate Copolymer, 30%ig in Wasser | |
| ²⁾Natriumpolystyrolsulfonat, Molekulargewicht ca. 130.000 | |

Der pH-Wert wird mit Aminomethylpropanol auf einen Wert im Bereich von 6 bis 8 eingestellt.

Konsistenz: Gelee, leicht verreibbar, gut auswaschbar

### Beispiel 2: Haargel

| | |
|---|---|
| 17 g | Structure® 3001 ¹⁾ |
| 4 g | Flexan® II ²⁾ |
| 2 g | Glycerin |
| 0,2 g | Parfüm |
| 0,2 g | PPG-1-PEG-9-Laurylglycolether |
| 0,4 g | Timiron® -Pigmente |
| Ad 100 g | wasser |

Der pH-Wert wird mit Aminomethylpropanol auf einen Wert im Bereich von 6 bis 8 eingestellt.

Konsistenz: Gelee, leicht verreibbar, gut auswaschbar

### Beispiel 3: Haargel

| | |
|---|---|
| 8 g | Structure® 3001 ¹⁾ |
| 7 g | Flexan® II ²⁾ |
| 10 g | 1,2-Propylenglykol |
| 0,2 g | Parfüm |
| 0,2 g | Oleth-20 |
| 0,065 g | Farbstoffe (D+C Violett No. 2, D+C Red No. 33, Colorona® -Pigmente) |
| Ad 100 g | Wasser |

Der pH-Wert wird mit Aminomethylpropanol auf einen Wert im Bereich von 6 bis 8 eingestellt.

Konsistenz: Gelee, leicht verreibbar, gut auswaschbar

### Beispiel 4: Haargel

| | |
|---|---|
| 15 g | Structure® 3001 ¹⁾ |
| 9 g | Flexan® II ²⁾ |
| 5 g | 1,2-Propylenglykol |
| 0,15 g | Parfüm |
| 0,15 g | PEG-40 hydriertes Rizinusöl |
| 0,3 g | Colorona®-Pigmente |
| q.s. | Anfärbefarbstoffe |
| Ad 100 g | Wasser |

Der pH-Wert wird mit Aminomethylpropanol auf einen Wert im Bereich von 6 bis 8 eingestellt.

Konsistenz: Gelee, leicht verreibbar, gut auswaschbar

### Beispiel 5: Haargel

| | |
|---|---|
| 10 g | Structure® 3001 ¹⁾ |
| 9 g | Flexan® 11 ²⁾ |
| 5 g | 1,2-Pentandiol |
| 0,15 g | Parfüm |
| 0,15 g | PPG-1-PEG-9-Laurylglycolether |
| 0,3 g | Timiron® -Pigmente |
| q.s. | Anfärbefarbstoffe |
| Ad 100 g | Wasser |

Der pH-Wert wird mit Aminomethylpropanol auf einen Wert im Bereich von 6 bis 8 eingestellt.

Konsistenz: Gelee, leicht verreibbar, gut auswaschbar

### Vergleichsversuche

| | A | B | C | D |
|---|---|---|---|---|
| Structure® 3001 ¹⁾ | 10 | 10 | 10 | 10 |
| Flexan® II ²⁾ | 5 | - | - | - |
| AMP-Acrylates/Allyl Methacrylate Copolymer | - | 5 | - | - |
| Amphomer ³⁾ | - | - | 5 | - |
| Vinylpyrrolidon/Vinylacetat Copolymer | - | - | - | 5 |
| 1,2-Propylenglykol | 3,6 | 3,6 | 3,6 | 3,6 |
| PEG-40 hydriertes Rizinusöl | 0,2 | 0,2 | 0,2 | 0,2 |
| PHB-Methylester | 0,4 | 0,4 | 0,4 | 0,4 |
| Wasser | Ad 100 | Ad 100 | Ad 100 | Ad 100 |

| | | | | |
|---|---|---|---|---|
| ¹⁾Acrylates/Ceteth-20 Itaconate Copolymer, 30%ig in Wasser | | | | |
| ²⁾Natriumpolystyrolsulfonat, Molekulargewicht ca. 130.000 | | | | |
| ³⁾ Octylacrylamide/Acrylates/Butylaminoethylmethacrylate Copolymer | | | | |

Die pH-Werte werden mit Aminomethylpropanol auf einen Wert im Bereich von 6 bis 8 eingestellt.

Das erfindungsgemäße Gel A und die nicht erfindungsgemäßen Gele B, C und D wurden hinsichtlich Konsistenz, Klebephase, Anfühlen auf der Hand und Verteilbarkeit qualitativ von 6 Testpersonen bewertet. Es wurde eine Bewertungsskala von 1 (sehr gut) bis 5 (sehr schlecht) zugrunde gelegt. Die angegebenen Zahlenwerte sind gerundete Mittelwerte. (sehr gut) bis 5 (sehr schlecht) zugrunde gelegt. Die angegebenen Zahlenwerte sind gerundete Mittelwerte.

| | A | B | C | D |
|---|---|---|---|---|
| Konsistenz | 2 | 4 | 3 | 3 |
| Klebephase | 2 | 3 | 3 | 3 |
| Anfühlen auf der Hand | 2 | 2 | 3 | 2 |
| Verteilbarkeit | 1,5 | 2 | 3 | 2 |

Das erfindungsgemäße Gel A zeigt bei der Klebephase einen deutlichen Vorteil und wird als einziges Gel mit gut bewertet. Auch bei der Verteilbarkeit zeigt es deutliche Vorteile und wird am besten bewertet.

### Beispiel 6 Stylinggel

- 8 g: Structure® 3001 (Acrylates/Ceteth-20 Itaconate Copolymer, 30%ig in Wasser)
- 5 g: Flexan II (Natriumpolystyrolsulfonat, Molekulargewicht ca. 130.000)
- 2 g: Structure® 2001 (Acrylates/Steareth-20 Itaconate Copolymer, 30%ig in Wasser)
- 1,75 g: Aminomethylpropanol (95%ig)
- 0,25 g: Carbomer
- Ad 100 g: Wasser

Das Gel mit einer Kombination von zwei Itaconatcopolymeren zeichnet sich durch eine besondere Langzeitstabilität der Konsistenz und rheologischen Eigenschaften aus.

## Patentansprüche

1. Haarbehandlungsmittel mit einem Gehalt an einer Kombination von
(A) mindestens einem Copolymeren aus einer ersten Monomerart, ausgewählt aus Itaconsäuremonoestern der allgemeinen Formel CH₂=C(COOR¹)CH₂COOR² wobei einer der Substituenten R¹ und R² für Wasserstoff und der andere für die Gruppe -(CH₂CH₂O)ₓ-R³ steht, x eine Zahl zwischen 1 und 100 bedeutet und R³ eine Alkylgruppe mit 8 bis 30 C-Atomen bedeutet und einer zweiten Monomerart, ausgewählt aus Acrylat-Monomeren und
(B) mindestens einem Polystyrolsulfonat mit einem Molekulargewicht im Bereich von 50.000 bis 200.000.

2. Mittel nach Anspruch 1, **dadurch gekennzeichnet, dass** das Copolymer (A) in einer Menge von 0,1 bis 30 Gew.% vorliegt.

3. Mittel nach einem der vorhergehenden Ansprüche, **dadurch gekennzeichnet, dass** das Polymer (B) in einer Menge von 0,1 bis 20 Gew.% vorliegt.

4. Mittel nach einem der vorhergehenden Ansprüche, **dadurch gekennzeichnet, dass** R³ für eine Alkylgruppe mit 12 bis 20 C-Atomen und x für eine Zahl von 10 bis 40 steht.

5. Mittel nach einem der vorhergehenden Ansprüche, **dadurch gekennzeichnet, dass** die zweite Monomerart des Copolymers (A) ausgewählt ist aus Acrylsäure, Methacrylsäure, Acrylsäurealkylestern und Methacrylsäurealkylestern, wobei die Alkylgruppen 1 bis 10 C-Atome aufweisen.

6. Mittel nach einem der vorhergehenden Ansprüche, **dadurch gekennzeichnet, dass** das Molekulargewicht des Polymers (B) im Bereich von 80.000 bis 180.000 liegt.

7. Mittel nach einem der vorhergehenden Ansprüche, **dadurch gekennzeichnet, dass** es im wesentlichen frei ist von kationischen Polymeren.

8. Mittel nach einem der vorhergehenden Ansprüche, **dadurch gekennzeichnet, dass** es zusätzlich mindestens einen Alkohol enthält ausgewählt aus einwertigen Alkoholen mit 1 bis 4 C-Atomen und mehrwertigen Alkoholen mit 2 bis 5 C-Atomen.

9. Mittel nach einem der vorhergehenden Ansprüche, **dadurch gekennzeichnet, dass** es zusätzlich mindestens ein weiteres, haarfestigendes nichtionisches, anionisches, zwitterionisches oder amphoteres Polymer enthält.

10. Mittel nach Anspruch 9, **dadurch gekennzeichnet, dass** das haarfestigende Polymer ausgewählt ist aus Polyvinylpyrrolidon, Vinylpyrrolidon/Vinylacetat Copolymer, Vinylacetat/Crotonsäure Copolymeren, Terpolymeren aus Vinylacetat, Crotonat und Vinylalkanoat, partialveresterten Copolymeren zwischen Vinylmethylether und Maleinsäureanhydrid, Copolymeren aus Acryl- oder Methacrylsäure mit Alkylacrylaten und/oder N-Alkylacrylamiden, Copolymeren aus Alkylacrylamid, Alkylaminoalkylmethacrylat und zwei oder mehr Monomeren ausgewählt aus Acrylsäure, Methacrylsäure oder deren C1- bis C4-Alkylestern, wobei mindestens eines der Monomere eine Säuregruppe aufweist.

11. Mittel nach einem der vorhergehenden Ansprüche, **dadurch gekennzeichnet, dass** es
(A) 0,5 bis 15 Gew.% eines Copolymeren aus einer ersten Monomerart, ausgewählt aus ethoxylierten Itaconsäuremonocetylestern mit einem Ethoxylierungsgrad von 10 bis 40 und einer zweiten Monomerart, ausgewählt aus Acrylat-Monomeren und
(B) 0,5 bis 7 Gew.% eines Polystyrolsulfonats mit einem Molekulargewicht von 80.000 bis 180.000 enthält,
wobei das Mittel in Form eines wässrigen Gels vorliegt und einen pH-Wert von 6 bis 9 aufweist.

12. Haarfärbemittel zum gleichzeitigen Festigen und temporären Färben von Haaren, **dadurch gekennzeichnet, dass** es eine Zusammensetzung gemäß einem der Ansprüche 1 bis 11 aufweist und zusätzlich mindestens ein haarfärbendes Pigment enthält.

13. Haarfärbegel nach Anspruch 12, **dadurch gekennzeichnet, dass** die Pigmente zu 0,01 bis 25 Gew.% enthalten sind und ausgewählt sind aus Metalloxiden, Ultramarin, Glanzpigmenten, Metalleffekt-Pigmenten, Perlglanzpigmenten, Fluoreszenzpigmenten, Phosphoreszenzpigmenten, Metallhydroxiden, Metalloxidhydraten, Mischphasenpigmenten, schwefelhaltigen Silicaten, Metallsulfiden, komplexen Metallcyaniden, Metallsulfaten, Metallchromaten, Metallmolybdaten, Bronzepigmenten, Carmine, Pigmenten auf Mica- oder Glimmerbasis, welche mit einem Metalloxid oder einem Metalloxychlorid sowie gegebenenfalls weiteren farbgebenden Stoffen wie Eisenoxiden, Eisenblau, Ultramarine, Carmine etc. beschichtet sind und/oder wobei die Farbe durch Variation der Schichtdicke bestimmt ist.

14. Mittel nach einem der vorhergehenden Ansprüche, **dadurch gekennzeichnet, dass** es mindestens ein erstes und mindestens ein zweites Copolymer aus jeweils einem Itaconsäuremonoester der Formel CH₂=C(COOH)CH₂COO-(CH₂CH₂O)ₓ-R³ und Acrylatmonomeren enthält, wobei x eine Zahl zwischen 10 und 40 und R³ des ersten Copolymers eine Cetylgruppe und R³ des zweiten Copolymers eine Stearylgruppe bedeuten.

## Claims

1. Hair treatment agent with a content of a combination of
(A) at least one copolymer of a first monomer type chosen from itaconic acid monoesters of the general formula CH₂=C(COOR¹)CH₂COOR², where one of the substituents R¹ and R² is hydrogen and the other is the group -(CH₂CH₂O)ₓ-R³, x is a number between 1 and 100 and R³ is an alkyl group having 8 to 30 carbon atoms, and a second monomer type chosen from acrylate monomers and
(B) at least one polystyrenesulphonate with a molecular weight in the range from 50 000 to 200 000.

2. Agent according to Claim 1, **characterized in that** the copolymer (A) is present in an amount of from 0.1 to 30% by weight.

3. Agent according to one of the preceding claims, **characterized in that** the polymer (B) is present in an amount of from 0.1 to 20% by weight.

4. Agent according to one of the preceding claims, **characterized in that** R³ is an alkyl group having 12 to 20 carbon atoms and x is a number from 10 to 40.

5. Agent according to one of the preceding claims, **characterized in that** the second monomer type of the copolymer (A) is chosen from acrylic acid, methacrylic acid, acrylic acid alkyl esters and methacrylic acid alkyl esters, where the alkyl groups have 1 to 10 carbon atoms.

6. Agent according to one of the preceding claims, **characterized in that** the molecular weight of the polymer (B) is in the range from 80 000 to 180 000.

7. Agent according to one of the preceding claims, **characterized in that** it is essentially free from cationic polymers.

8. Agent according to one of the preceding claims, **characterized in that** it additionally comprises at least one alcohol chosen from monohydric alcohols having 1 to 4 carbon atoms and polyhydric alcohols having 2 to 5 carbon atoms.

9. Agent according to one of the preceding claims, **characterized in that** it additionally comprises at least one further, hair-setting nonionic, anionic, zwitterionic or amphoteric polymer.

10. Agent according to Claim 9, **characterized in that** the hair-setting polymer is chosen from polyvinylpyrrolidone, vinylpyrrolidone/vinyl acetate copolymer, vinyl acetate/crotonic acid copolymers, terpolymers of vinyl acetate, crotonate and vinyl alkanoate, partially esterified copolymers between vinyl methyl ether and maleic anhydride, copolymers of acrylic or methacrylic acid with alkyl acrylates and/or N-alkylacrylamides, copolymers of alkylacrylamide, alkylaminoalkyl methacrylate and two or more monomers chosen from acrylic acid, methacrylic acid or C1 to C4 alkyl esters thereof, where at least one of the monomers has an acid group.

11. Agent according to one of the preceding claims, **characterized in that** it comprises
(A) 0.5 to 15% by weight of a copolymer of a first monomer type chosen from ethoxylated itaconic acid monocetyl esters with a degree of ethoxylation of from 10 to 40 and a second monomer type chosen from acrylate monomers and
(B) 0.5 to 7% by weight of a polystyrenesulphonate with a molecular weight of from 80 000 to 180 000,
where the agent is in the form of an aqueous gel and has a pH of from 6 to 9.

12. Hair colorant for the simultaneous setting and temporary colouring of hair, **characterized in that** it has a composition according to one of Claims 1 to 11 and additionally comprises at least one hair-colouring pigment.

13. Hair-colouring gel according to Claim 12, **characterized in that** the pigments are present to 0.01 to 25% by weight and are chosen from metal oxides, ultramarine, lustre pigments, metal effect pigments, pearlescent pigments, fluorescent pigments, phosphorescent pigments, metal hydroxides, metal oxide hydrates, mixed phase pigments, sulphur-containing silicates, metal sulphides, complex metal cyanides, metal sulphates, metal chromates, metal molybdates, bronze pigments, carmines, pigments based on mica which are coated with a metal oxide or a metal oxychloride, and optionally further colour-imparting substances such as iron oxides, iron blue, ultramarines, carmines, etc. and/or where the colour is determined by varying the layer thickness.

14. Agent according to one of the preceding claims, **characterized in that** it comprises at least one first and at least one second copolymer of in each case an itaconic acid monoester of the formula CH₂=C(COOH)CH₂COO-(CH₂CH₂O)ₓ-R³ and acrylate monomers, where x is a number between 10 and 40 and R³ of the first copolymer is a cetyl group and R³ of the second copolymer is a stearyl group.

## Revendications

1. Composition de traitement capillaire ayant un teneur en une association de
(A) au moins un copolymère à base d'un premier type de monomère, choisi parmi les monoesters d'acide itaconique de formule générale CH₂=C(COOR¹)CH₂COOR², dans laquelle l'un des substituants R¹ et R² représente un atome d'hydrogène et l'autre représente le groupe -(CH₂CH₂O)ₓ-R³, x représente un nombre compris entre 1 et 100, et R³ représente un groupe alkyle ayant de 8 à 30 atomes de carbone, et d'un second type de monomère choisi parmi les monomères acrylate et
(B) au moins un polystyrènesulfonate ayant une masse moléculaire dans la plage de 50 000 à 200 000.

2. Composition selon la revendication 1, **caractérisée en ce que** le copolymère (A) est présent en une quantité de 0,1 à 30 % en poids.

3. Composition selon l'une quelconque des revendications précédentes, **caractérisée en ce que** le polymère (B) est présent en une quantité de 0,1 à 20 % en poids.

4. Composition selon l'une quelconque des revendications précédentes, **caractérisée en ce que** R³ représente un groupe alkyle ayant de 12 à 20 atomes de carbone et x représente un nombre allant de 10 à 40.

5. Composition selon l'une quelconque des revendications précédentes, **caractérisée en ce que** le second type de monomère du copolymère (A) est choisi parmi l'acide acrylique, l'acide méthacrylique, des esters alkyliques d'acide acrylique et des esters alkyliques d'acide méthacrylique, les groupes alkyle ayant de 1 à 10 atomes de carbone.

6. Composition selon l'une quelconque des revendications précédentes, **caractérisée en ce que** la masse moléculaire du polymère (B) se situe dans la plage allant de 80 000 à 180 000.

7. Composition selon l'une quelconque des revendications précédentes, **caractérisée en ce qu'**elle, est pratiquement exemple de polymères cationiques.

8. Composition selon l'une quelconque des revendications précédentes, **caractérisée en ce qu'**additionnellement elle contient au moins un alcool choisi parmi les alcools monohydriques ayant de 1 à 4 atomes de carbone et les alcools polyhydriques ayant de 2 à 5 atomes de carbone.

9. Composition selon l'une quelconque des revendications précédentes, **caractérisée en ce qu'**en outre elle contient au moins un autre polymère non ionique, anionique, zwitterionique ou amphotère, fixant les cheveux.

10. Composition selon la revendication 9, **caractérisée en ce que** le polymère fixant les cheveux est choisi parmi la polyvinylpyrrolidone, un copolymère vinylpyrrolidone/acétate de vinyle, des copolymères acétate de vinyle/acide crotonique, des terpolymères d'acétate de vinyle, crotonate et alcanoate de vinyle, des copolymères entre l'oxyde de vinyle et de méthyle et l'anhydride maléique, partiellement estérifiés, des copolymères d'acide acrylique ou méthacrylique avec des acrylates d'alkyle et/ou des N-alkylacrylamides, des copolymères d'alkylacrylamide, méthacrylate d'alkylaminoalkyle et de deux ou plus de deux monomères choisis parmi l'acide acrylique, l'acide méthacrylique ou leurs esters alkyliques en C₁-C₄, au moins l'un des monomères comportant un groupe acide.

11. Composition selon l'une quelconque des revendications précédentes, **caractérisée en ce qu'**elle comporte
(A) 0,5 à 15 % en poids d'un copolymère d'un premier type de monomère choisi parmi des itaconates de monocétyle éthoxylés ayant un degré d'éthoxylation de 10 à 40 et d'un second type de monomère choisi parmi des monomères acrylate et
(B) 0,5 à 7 % en poids d'un polystyrènesulfonate ayant une masse moléculaire de 80 000 à 180 000,
la composition se trouvant sous forme d'un gel aqueux ayant un pH de 6 à 9.

12. Produit de teinture pour cheveux pour la coloration temporaire et le fixage simultané des cheveux, **caractérisé en ce qu'**il a une composition selon l'une quelconque des revendications 1 à 11 et additionnellement contient au moins un pigment colorant les cheveux.

13. Gel colorant pour cheveux selon la revendication 12, **caractérisée en ce que** les pigments sont contenus à raison de 0,01 à 25 % en poids et sont choisis parmi des oxydes métalliques, l'outremer, des pigments brillants, des pigments à effet métallique, des pigments nacrés, des pigments fluorescents, des pigments phosphorescents, des hydroxydes de métaux, des hydrates de métaux, des pigments en phases mixtes, des silicates soufrés, des sulfures métalliques, des cyanures métalliques complexes, des sulfates métalliques, des chromates métalliques, des molybdates métalliques, des pigments bronze, des carmins, des pigments à base de mica qui sont enrobés d'un oxyde métallique ou d'un oxychlorure métallique ainsi qu'éventuellement d'autres substances colorantes telles que des oxydes de fer, le bleu de Prusse, les outremers, les carmins, etc., la couleur étant déterminée par variation de l'épaisseur de couche.

14. Composition selon l'une quelconque des revendications précédentes, **caractérisée en ce qu'**elle contient au moins un premier et au moins un second copolymère à base respectivement d'un monoester d'acide itaconique de formule CH₂=C(COOH)CH₂COO-(CH₂CH₂O)ₓ-R³ et de monomères d'acrylate, x représentant un nombre compris entre 10 et 40 et R³ du premier copolymère représentant un groupe cétyle et R³ du second copolymère représentant un groupe stéaryle.
